# EUROPÄISCHE PATENTSCHRIFT

(11) **EP 2 625 158 B1**
(45) Veröffentlichungstag und Bekanntmachung des Hinweises auf die Patenterteilung: **13.08.2014**
(21) Anmeldenummer: 11767683.3
(22) Anmeldetag: 05.10.2011
(51) Int. Cl.: C07C 45/28, C07C 45/62, C07C 49/385, C07C 49/587

(54) **VERFAHREN ZUR HERSTELLUNG VON KETONEN, INSBESONDERE MAKROCYCLISCHER KETONE**
PROCESS FOR PREPARING KETONES, IN PARTICULAR MACROCYCLIC KETONES
PROCÉDÉ DE PRODUCTION DE CÉTONES, EN PARTICULIER DE CÉTONES MACROCYCLIQUES

(30) Priorität: 07.10.2010 EP 10186917
(43) Veröffentlichungstag der Anmeldung: 14.08.2013
(73) Patentinhaber: BASF SE, 67056 Ludwigshafen (DE)
(72) Erfinder: SCHELPER, Michael, 67065 Ludwigshafen (DE); STOCK, Christoph, 67158 Ellerstadt (DE); EBEL, Klaus, 68623 Lampertheim (DE); TELES, Joaquim Henrique, 67165 Waldsee (DE); PELZER, Ralf, 37699 Fürstenberg (DE)
(86) Internationale Anmeldenummer: PCT/EP2011/067409
(87) Internationale Veröffentlichungsnummer: WO 2012/045786

(56) Entgegenhaltungen:
- WO-A1-2008/000756
- WO-A1-2010/076182
- DE-A1- 1 668 054
- C. FEHR; G. OHLOFF: "A New alpha-beta-Enone - Alkynone Fragmentation. Syntheses of Exaltone and (+/-)-Muscone", HELV. CHIM. ACTA, Bd. 62, 1979, Seiten 2655-2660, XP002663600, ISSN: 0018-019X in der Anmeldung erwähnt

## Beschreibung

Gesättigte makrocyclische Ketone mit 14- bis 18-gliedrigen Ringen, wie z. B. Muscon (3-Methylcyclopentadecanon), sind begehrte Riech- oder Aromastoffe. Da die Verbindungen aus natürlichen Quellen nur in geringen Mengen zur Verfügung stehen, ist ihre synthetische Herstellung Gegenstand umfangreicher Untersuchungen.

Die Herstellung von Muscon aus 14-Methyl-bicyclo[10.3.0]pentadecen[1(12)] ist seit langem bekannt. So beschreiben die CH-503 680 und US-A 3,778,483 ein Verfahren zur Herstellung von Muscon, bei dem man 14-Methyl-bicyclo[10.3.0]pentadecen[1(12)] unter Spaltung der zentralen Doppelbindung mit Ozon oxidiert, eine Ketogruppe des erhaltenen Diketons zum Alkohol reduziert und das nach Wasserabspaltung erhaltene ungesättigte makrocyclische Keton zum Muscon hydriert.

V. Rautenstrauch et al., Helv. Chim. Acta Vol. 73, (1990) S. 896) beschreiben eine Synthese von Muscon, die von 2-(2'-Methylprop-2'-enyl)cyclododecan-1-on ausgeht und über (3aRS,13aSR)-3a,4,5,6,7,8,9,10,11,12,13,13a-Dodecahydro-2-methyl-1H-cyclopentacyclododecen-13a-ol verläuft. Weitere Synthesen von Muscon sind in der DE 1 668 054 und Helv. Chim. Acta Vol. 62, (1979) S. 2657 beschrieben.

Die bekannten Verfahren sind jedoch aufgrund der verwendeten Reagenzien, ihrer Komplexität und/oder der erzielten Ausbeuten nicht zufriedenstellend. Der Erfindung liegt die Aufgabe zugrunde, ein Verfahren anzugeben, nach dem bestimmte gesättigte Ketone, insbesondere gesättigte makrocyclische Ketone, wie Muscon, technisch auf einfache Weise erhalten werden können.

Die Oxidation von Olefinen mit N₂O zu einem Aldehyd oder einem Keton ist eine seit langem bekannte Reaktion und ist beispielsweise in der GB 649,680 beschrieben. Die WO 2008/000756 beschreibt ein Verfahren zur Herstellung eines cyclischen Ketons mit 7 bis 16 C-Atomen, wobei ein cyclisches Alken mit 7 bis 16 C-Atomen mit mindestens einer C=C-Doppelbindung mittels Distickstoffmonoxid oxidiert wird.

Eine Bindungsspaltung der C=C-Doppelbindung tritt bei dieser Reaktion üblicherweise nicht oder nur in untergeordnetem Maße auf. Die Ausnahme bilden einige gespannte Ringsysteme wie Bicyclohepten oder Inden; siehe E.V. Starokon, Adv. Synth. Catal. 2004, 346, 268-274. Die Oxidation tetrasubstituierter Olefine mit N₂O wurde bislang nur am Beispiel des Tetramethylethens beschrieben. Dort wurde keine Spaltung der C=C-Doppelbindung beobachtet, siehe Bridson-Jones et al., JCS (1951), 3009.

Die Erfindung betrifft ein Verfahren zur Herstellung von Ketonen der Formel II, 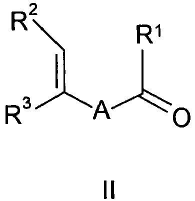 worin
- A: für C₂-C₁₂-Alkandiyl steht, das gegebenenfalls durch 1 bis 5 C₁-C₆-Alkylgruppen substituiert ist,
- R¹ und R²: unabhängig voneinander für C₁-C₆-Alkyl stehen, oder R¹ und R² gemeinsam für C₃-C₁₀-Alkandiyl steht, das gegebenenfalls durch 1 bis 5 C₁-C₆-Alkylgruppen substituiert ist, und
- R³: für Wasserstoff oder C₁-C₆-Alkyl steht;
wobei man
(a) ein cyclisches Olefin der Formel I 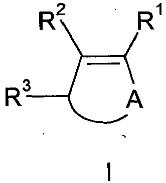 mit Distickstoffmonoxid (N₂O) zum Keton der Formel II umsetzt.
   Die Erfindung betrifft weiterhin ein Verfahren, bei dem man außerdem
(b) das Keton der Formel II zum gesättigten Keton der Formel III hydriert

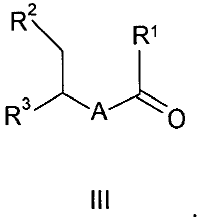

Für die Zwecke der vorliegenden Anmeldung bedeutet Alkandiyl vorzugsweise Alkan-(α,ω)-diyl.

In den Formeln I, II und III steht A für C₂-C₁₂-Alkandiyl, vorzugsweise C₂-C₉-Alkandiyl, wie Ethan-1,2-diyl, Propan-1,3-diyl, Butan-1,4-diyl, Pentan-1,5-diyl, Hexan-1,6-diyl, Heptan-1,7-diyl, Octan-1,8-diyl oder Nonan-1,9-diyl. Vorzugsweise steht A für C₃-C₉-Alkandiyl. Das Alkandiyl ist gegebenenfalls durch 1 bis 5 C₁-C₆-Alkylgruppen substituiert, wie Methyl, Ethyl, Propyl, Isopropyl, Butyl, sec-Butyl, tert-Butyl, Pentyl oder Hexyl, insbesondere Methyl.

R¹ und R² stehen unabhängig voneinander für C₁-C₆-Alkyl, wie Methyl, Ethyl, Propyl, Isopropyl, Butyl, sec-Butyl, tert-Butyl, Pentyl oder Hexyl.

Alternativ stehen R¹ und R² gemeinsam für C₃-C₁₀-Alkandiyl, wie Propan-1,3-diyl, Butan-1,4-diyl, Pentan-1,5-diyl, Hexan-1,6-diyl, Heptan-1,7-diyl, Octan-1,8-diyl, Nonan-1,9-diyl oder Decan-1-10-diyl. Vorzugsweise stehen R¹ und R² gemeinsam für C₃-Alkandiyl oder C₄-Alkandiyl. Sofern R¹ und R² gemeinsam für Alkandiyl stehen, handelt es sich bei dem cyclischen Olefin eine bicyclische Verbindung, wobei die beiden Ringe über eine gemeinsame C=C-Doppelbindung verfügen. Das Alkandiyl ist gegebenenfalls durch 1 bis 5 C₁-C₆-Alkylgruppen substituiert, wie Methyl, Ethyl, Propyl, Isopropyl, Butyl, sec-Butyl, tert-Butyl, Pentyl oder Hexyl, insbesondere Methyl.

R³ steht für Wasserstoff oder C₁-C₆-Alkyl, wie Methyl, Ethyl, Propyl, Isopropyl, Butyl, sec-Butyl, tert-Butyl, Pentyl oder Hexyl.

Ein bevorzugtes gesättigtes Keton der Formel III, das nach dem erfindungsgemäßen Verfahren erhalten werden kann, ist (±)-3-Methylcyclopentadecanon (Muscon, racemisches Muscon oder rac-Muscon) der Formel IIIa

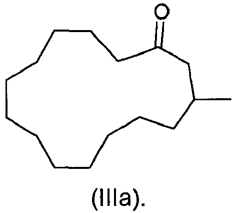

Hierfür setzt man 14-Methyl-bicyclo[10.3.0]pentadecen[1(12)] (2,3,4,5,6,7,8,9,10, 11,12,13-Dodecahydro-2-methyl-1H-cyclopentacyclododecen) der Formel la als cyclisches Olefin der Formel I ein

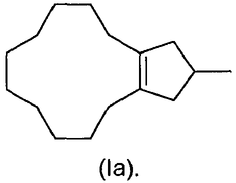

Die Verbindung la und ihre Herstellung sind bekannt. la ist vorteilhaft auf folgende Weise erhältlich: Man setzt nach dem Verfahren gemäß der DE 29 16 418 unter den Bedingungen einer Phasentransfer-Reaktion Cyclododecanon mit einem Methallylhalogenid um. Das erhaltene 2-(2'-Methylen-1'-propyl)cyclododecanon kann gemäß der US 4,967,033 unter sauren Bedingungen in der Gasphase cyclisiert werden. Dabei wird Methylbicyclo[10.3.0]pentadecadien in Form verschiedener Doppelbindungsisomere erhalten. Dieses Gemisch wird anschließend einer Palladium-katalysierten Hydrierung unterworfen, wobei im Wesentlichen nur eine Doppelbindung hydriert wird und gleichzeitig eine Doppelbindungsisomerisierung stattfindet, so dass selektiv im Wesentlichen la erhalten wird, vgl. Helvetica Chimica Acta 92 (9), 2009, 1782-1799 und DE 29 16 418 C2.

Andere gesättigte Ketone, die nach dem erfindungsgemäßen Verfahren hergestellt werden können, sind z. B. Cyclopentadecanon (Exalton) oder Cyclohexadecanon.

Im Schritt (a) des erfindungsgemäßen Verfahrens erfolgt vermutlich eine Cycloaddition von Distickstoffmonoxid zum Addukt der Formel IV, das unter Stickstoffeliminierung zum Keton der Formel II umlagert.

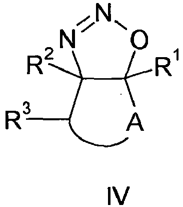

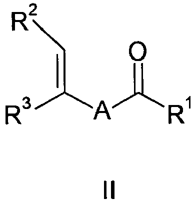

Das Keton der Formel II verfügt über eine Doppelbindung, die in einer unerwünschten Nebenreaktion erneut mit Distickstoffmonoxid unter Bildung eines Diketons reagieren kann. Es ist daher bevorzugt, den Umsatz im Schritt (a) gering zu halten, d.h. die Umsetzung des cyclischen Olefins der Formel I mit Distickstoffmonoxid nur bis zu einem Teilumsatz zu betreiben. Der Teilumsatz beträgt vorzugsweise 20 Mol-% oder weniger, insbesondere 5 bis 18 Mol-%, bezogen auf die anfängliche Menge an cyclischem Olefin der Formel I (bei diskontinuierlicher Verfahrensführung) bzw. bezogen auf das cyclische Olefin der Formel I im Zulauf (bei kontinuierlicher Verfahrensführung). Man erhält so ein erstes Gemisch, das nicht umgesetztes cyclisches Olefin der Formel I und Keton der Formel II umfasst. Das erste Gemisch kann auf verschiedene Weise aufgearbeitet werden.

Zweckmäßigerweise kann man das erste Gemisch ohne vorhergehende Auftrennung einer Hydrierung unterwerfen.

Eine bevorzugte Ausführungsform betrifft daher ein Verfahren, bei dem man
(i) das cyclische Olefin der Formel I unter Teilumsatz mit Distickstoffmonoxid zu einem ersten Gemisch umsetzt, das nicht umgesetztes cyclisches Olefin der Formel I und Keton der Formel II umfasst, und
(ii) das erste Gemisch unter Bedingungen hydriert, unter denen das Keton der Formel II zum gesättigten Keton der Formel III hydriert wird und das cyclische Olefin der Formel I im Wesentlichen nicht hydriert wird, wobei man ein zweites Gemisch erhält, das nicht umgesetztes cyclisches Olefin der Formel I und gesättigtes Keton der Formel III umfasst.

Die C=C-Doppelbindung im ungesättigten Keton der Formel II ist disubstituiert (für R³ = H) oder trisubstituiert (für R³ = C₁-C₆-Alkyl) und daher im Allgemeinen reaktiver als die tetrasubstituierte C=C-Doppelbindung im cyclischen Olefin der Formel I. Daher gelingt unter geeigneten Reaktionsbedingungen die im Wesentlichen selektive Hydrierung des ungesättigten Ketons der Formel II in der Regel problemlos. Für die Zwecke des vorliegenden Verfahrens ist es im Allgemeinen ausreichend, dass mehr als 90 Mol-% der C=C-Doppelbindung im ungesättigten Keton der Formel II und weniger als 10 Mol-% der tetrasubstituierten C=C-Doppelbindung im cyclischen Olefin der Formel I hydriert werden. Die an sich unerwünschte Hydrierung einer geringen Menge des cyclischen Olefins der Formel I kann toleriert werden, da das Hydrierungsprodukt des cyclischen Olefins der Formel I nicht reaktiv gegenüber Distickstoffmonoxid ist und sich vom gewünschten Reaktionsprodukt durch Destillation leicht abtrennen lässt.

Vorzugsweise führt man zumindest einen Teil des bei der Hydrierung erhaltenen zweiten Gemisches zum Schritt (i) zurück und unterwirft es einer erneuten Umsetzung mit Distickstoffmonoxid und hydriert das dabei erhaltene Gemisch erneut. Diese Vorgehensweise wird so oft wiederholt, bis ein gewünschter Gesamtumsatz des cyclischen Olefins der Formel I erreicht ist bzw. bis der gewünschte Gehalt an gesättigtem Keton der Formel III im Produktgemisch erreicht ist. Abschließend erfolgt eine Aufarbeitung des Produktgemisches.

Alternativ kann man das erfindungsgemäße Verfahren auch kontinuierlich ausführen, wobei man einen Teil des zweiten Gemisches mit frischem cyclischen Olefin der Formel I versetzt und zum Schritt (i) zurückführt und einen Teil des zweiten Gemisches abzieht und gesättigtes Keton der Formel III daraus isoliert.

Eine andere zweckmäßige Ausführungsform betrifft ein Verfahren, wobei man
(i) das cyclische Olefin der Formel I unter Teilumsatz mit Distickstoffmonoxid zu einem ersten Gemisch umsetzt, das nicht umgesetztes cyclisches Olefin der Formel I und Keton der Formel II umfasst, und
(ii) aus dem ersten Gemisch nicht umgesetztes cyclisches Olefin der Formel I ab-trennt, wobei man einen Rückstand erhält, der ungesättigtes Keton der Formel II umfasst, und den Rückstand hydriert, wobei man einen hydrierten Rückstand erhält, der gesättigtes Keton der Formel III umfasst.

Die Abtrennung des nicht umgesetzten cyclischen Olefins der Formel I erfolgt vorzugsweise durch Destillation, wobei der Rückstand, der das ungesättigte Keton der Formel II umfasst, als Sumpfrückstand anfällt. Vorzugsweise führt man das abgetrennte nicht umgesetzte cyclische Olefin zumindest teilweise in den Schritt (i) zurück, gegebenenfalls zusammen mit frischem cyclischen Olefin der Formel I. Aus dem hydrierten Rückstand kann dann das gesättigtes Keton der Formel III isoliert werden.

Im Allgemeinen ist bei der erfindungsgemäßen Umsetzung mit Distickstoffmonoxid der Zusatz eines Lösungsmittels oder Verdünnungsmittel nicht notwendig. Zweckmäßigerweise führt man daher die die Schritte (a) und (b) in Abwesenheit eines externen Lösungsmittels durch. Externe Lösungsmittel sind Lösungsmittel, die kein Edukt, Produkt oder Nebenprodukt des erfindungsgemäßen Verfahrens sind.

Die Temperaturen bei der Umsetzung des cyclischen Olefins der Formel I mit Distickstoffmonoxid liegen im Allgemeinen im Bereich von 140 bis 350 °C, vorzugsweise im Bereich von 160 bis 275 °C oder in einem Bereich von 200 bis 310 °C und besonders bevorzugt im Bereich von 180 bis 250 °C oder von 250 bis 300 °C.

Im Rahmen des erfindungsgemäßen Verfahrens ist es möglich, die Reaktion bei zwei oder mehr Temperaturen beziehungsweise in zwei oder mehr Temperaturbereichen durchzuführen, die jeweils in den oben angegebenen Grenzen liegen. Temperaturänderungen im Laufe der Umsetzung können kontinuierlich oder auch diskontinuierlich vollzogen werden.

Die Drücke bei der Umsetzung des cyclischen Olefins der Formel I mit Distickstoffmonoxid liegen bevorzugt höher als der Eigendruck des Edukt- bzw. Produktgemisches bei der gewählten Reaktionstemperatur oder den gewählten Reaktionstemperaturen. Die Umsetzung erfolgt vorzugsweise unter einem Druck, unter dem Distickstoffmonoxid in kondensierter Phase, d. h. flüssiger oder überkritischer Phase, vorliegt. Die Drücke liegen bevorzugt im Bereich von 1 bis 1000 bar, stärker bevorzugt im Bereich von 40 bis 325 bar und besonders bevorzugt im Bereich von 50 bis 200 bar.

Im Rahmen des erfindungsgemäßen Verfahrens ist es möglich, die Reaktion bei zwei oder mehr Drücken beziehungsweise in zwei oder mehr Druckbereichen durchzuführen, die jeweils in den oben angegebenen Grenzen liegen. Druckänderungen im Laufe der Umsetzung können kontinuierlich oder auch diskontinuierlich vollzogen werden.

Die für die Umsetzung einsetzbaren Reaktoren unterliegen keinen besonderen Beschränkungen. Insbesondere kann die Umsetzung in Batch-Fahrweise oder in kontinuierlicher Fahrweise erfolgen. Demgemäß können beispielsweise als Reaktoren mindestens ein CSTR (Continuous Stirred Tank Reactor) mit mindestens einem internen und/oder mindestens einem externen Wärmetauscher, mindestens ein Rohrreaktor oder mindestens ein Schlaufenreaktor eingesetzt werden. Ebenso ist es möglich, mindestens einen dieser Reaktoren so auszugestalten, dass er mindestens zwei unterschiedliche Zonen aufweist. Solche Zonen können sich beispielsweise in Reaktionsbedingungen wie beispielsweise der Temperatur oder dem Druck und/oder in der Geometrie der Zone wie beispielsweise dem Volumen oder dem Querschnitt unterscheiden. Wird die Umsetzung in zwei oder mehr Reaktoren durchgeführt, können zwei oder mehr gleiche Reaktortypen oder mindestens zwei verschiedene Reaktortypen eingesetzt werden.

Das Distickstoffmonoxid wird zweckmäßigerweise als Flüssigkeit mit einer Dosierpumpe in den Reaktor eingetragen. Denkbar ist es aber auch, gasförmiges Distickstoffmonoxid vorab unter Bedingungen in dem cyclischen Olefin der Formel I oder einem das Olefin enthaltenden Einsatzgemisch zu lösen, unter denen keine nennenswerte Umsetzung erfolgt, insbesondere bei einer ausreichend niedrigen Temperatur, und dann dieses Gemisch, z.B. mit einer Dosierpumpe, in den Reaktor zu pumpen.

Gemäß einer bevorzugten Ausführungsform wird die Umsetzung mit Distickstoffmonoxid in einem einzigen Reaktor durchgeführt. Gemäß einer weiteren bevorzugten Ausführungsform wird die Umsetzung mit Distickstoffmonoxid in einem einzigen Rohrreaktor durchgeführt.

Die Verweilzeit des Reaktionsgemisches in dem mindestens einen Reaktor liegt im Allgemeinen im Bereich von bis zu 20 h, bevorzugt im Bereich von 0,1 bis 20 Stunden, weiter bevorzugt im Bereich von 0,2 bis 15 Stunden und besonders bevorzugt im Bereich von 0,25 bis 10 h.

Das Molverhältnis von Distickstoffmonoxid zum cyclischen Olefin der Formel I liegt im Allgemeinen im Bereich von 0,05 bis 4, bevorzugt im Bereich von 0,06 bis 1, stärker bevorzugt im Bereich von 0,07 bis 0,5 und besonders bevorzugt im Bereich von 0,1 bis 0,4.

Für die Hydrierung des Ketons der Formel II können beliebige geeignete Katalysatoren eingesetzt werden. Insbesondere sind homogene und/oder heterogene Katalysatoren einsetzbar. Die Hydrierung erfolgt jedoch vorzugsweise in Gegenwart eines heterogenen Hydrierkatalysators.

Bevorzugt enthalten die einsetzbaren Katalysatoren mindestens ein Metall aus der 7., der 8., der 9., der 10. oder der 11. Nebengruppe des Periodensystems der Elemente. Weiter bevorzugt enthalten die einsetzbaren Katalysatoren mindestens ein Element, ausgewählt aus der Gruppe bestehend aus Re, Fe, Ru, Co, Rh, Ir, Ni, Pd, Pt, Cu und Au.

Insbesondere bevorzugt enthalten die einsetzbaren Katalysatoren mindestens ein Element, ausgewählt aus der Gruppe bestehend aus Fe, Ni, Pd, Pt und Cu. Besonders bevorzugt enthalten die eingesetzten Katalysatoren Pd.

Im erfindungsgemäßen Verfahren bevorzugt eingesetzte homogene Katalysatoren enthalten mindestens ein Element der 8., 9. oder 10. Nebengruppe. Weiter bevorzugt sind homogenen Katalysatoren, die Ru, Rh, Ir und/oder Ni enthalten. Beispielsweise sind hierbei etwa RhCl(TTP)₃ (TTP = Ph₂P(CH₂)₂PPh(CH₂)₂PPh₂) oder Ru₄H₄(CO)₁₂ zu nennen. Besonders bevorzugt sind solche homogenen Katalysatoren, die Ru enthalten. Beispielsweise werden homogene Katalysatoren eingesetzt, wie sie in der US 5,180,870, US 5,321, 176, US 5,177,278, US 3,804,914, US 5,210,349 US 5,128,296, US B 316,917 und in D. R. Fahey in J. Org. Chem. 38 (1973) S. 80-87 beschrieben sind, auf die vollinhaltlich verwiesen wird. Solche Katalysatoren sind etwa (TPP)₂(CO)₃Ru, [Ru(CO)₄]₃, (TPP)₂Ru(CO)₂Cl₂, (TPP)₃(CO)RuH₂, (TPP)₂(CO)₂RuH₂, (TPP)₂(CO)₂RuCIH oder (TPP)₃(CO)RuCl₂ (TPP = Triphenylphosphin).

Insbesondere bevorzugt wird im Rahmen des erfindungsgemäßen Verfahrens mindestens ein heterogener Katalysator eingesetzt, wobei mindestens eines der obenstehend genannten Metalle als Metall als solches, als Raney-Katalysator und/oder aufgebracht auf einen üblichen Träger eingesetzt werden kann. Bevorzugte Trägermaterialien sind etwa Aktivkohlen oder Oxide wie beispielsweise Aluminiumoxide, Siliciumoxide, Titanoxide oder Zirkonoxide. Ebenso sind unter anderem als Trägermaterialien Bentonite zu nennen. Werden zwei oder mehr Metalle eingesetzt, so können diese separat oder als Legierung vorliegen. Hierbei ist es möglich, mindestens ein Metall als solches und mindestens ein anderes Metall als Raney-Katalysator oder mindestens ein Metall als solches und mindestens ein anderes Metall, aufgebracht auf mindestens einen Träger, oder mindestens ein Metall als Raney-Katalysator und mindestens ein anderes Metall, aufgebracht auf mindestens einen Träger, oder mindestens ein Metall als solches und mindestens ein anderes Metall als Raney-Katalysator und mindestens ein anderes Metall, aufgebracht auf mindestens einen Träger, einzusetzen.

Die im erfindungsgemäßen Verfahren eingesetzten Katalysatoren können beispielsweise auch so genannte Fällungskatalysatoren sein. Solche Katalysatoren können hergestellt werden, indem man ihre katalytisch aktiven Komponenten aus deren Salzlösungen, insbesondere aus den Lösungen von deren Nitraten und/oder Acetaten, beispielsweise durch Zugabe von Lösungen von Alkalimetall- und/oder Erdalkalimetallhydroxid- und/oder Carbonat-Lösungen, beispielsweise schwerlöslichen Hydroxiden, Oxidhydraten, basischen Salzen oder Carbonaten ausfällt, die erhaltenen Niederschläge anschließend trocknet und diese dann durch Calcinierung bei im Allgemeinen 300 bis 700 °C, insbesondere 400 bis 600 °C in die entsprechenden Oxide, Mischoxide und/oder gemischtvalenten Oxide umwandelt, die durch eine Behandlung mit Wasserstoff oder mit Wasserstoff enthaltenden Gasen im Bereich von im Allgemeinen 50 bis 700 °C, insbesondere 100 bis 400 °C zu den betreffenden Metallen und/oder oxidischen Verbindungen niederer Oxidationsstufe reduziert und in die eigentliche katalytisch aktive Form überführt werden. Dabei wird in der Regel so lange reduziert, bis kein Wasser mehr gebildet wird. Bei der Herstellung von Fällungskatalysatoren, die ein Trägermaterial enthalten, kann die Fällung der katalytisch aktiven Komponenten in Gegenwart des betreffenden Trägermaterials erfolgen. Die katalytisch aktiven Komponenten können vorteilhaft gleichzeitig mit dem Trägermaterial aus den betreffenden Salzlösungen gefällt werden.

Bevorzugt werden im erfindungsgemäßen Verfahren Hydrierkatalysatoren eingesetzt, welche die die Hydrierung katalysierenden Metalle oder Metallverbindungen auf einem Trägermaterial abgeschieden enthalten.

Neben den oben genannten Fällungskatalysatoren, die außer den katalytisch aktiven Komponenten noch zusätzlich ein Trägermaterial enthalten, eignen sich für das erfindungsgemäße Verfahren im Allgemeinen solche Trägermaterialien, bei denen die katalytisch-hydrierend wirkende Komponente beispielsweise durch Imprägnierung auf ein Trägermaterial aufgebracht worden ist.

Die Art des Aufbringens des katalytisch aktiven Metalls auf den Träger ist in der Regel nicht kritisch und kann auf verschiedenerlei Art und Weise bewerkstelligt werden. Die katalytisch aktiven Metalle können auf diesen Trägermaterialien beispielsweise durch Tränkung mit Lösungen oder Suspensionen der Salze oder Oxide der betreffenden Elemente, Trocknung und anschließende Reduktion der Metallverbindungen zu den betreffenden Metallen oder Verbindungen niederer Oxidationsstufe mittels eines Reduktionsmittels, vorzugsweise mit Wasserstoff oder komplexen Hydriden, aufgebracht werden. Eine andere Möglichkeit zur Aufbringung der katalytisch aktiven Metalle auf diese Träger besteht darin, die Träger mit Lösungen thermisch leicht zersetzbarer Salze, beispielsweise mit Nitraten oder thermisch leicht zersetzbaren Komplexverbindungen, beispielsweise Carbonyl- oder Hydrido-Komplexen der katalytisch aktiven Metalle, zu imprägnieren und den so getränkten Träger zur thermischen Zersetzung der adsorbierten Metallverbindungen auf Temperaturen im Bereich von 300 bis 600 °C zu erhitzen. Diese thermische Zersetzung wird vorzugsweise unter einer Schutzgasatmosphäre vorgenommen. Geeignete Schutzgase sind beispielsweise Stickstoff, Kohlendioxid, Wasserstoff oder die Edelgase. Weiterhin können die katalytisch aktiven Metalle auf dem Katalysatorträger durch Aufdampfen oder durch Flammspritzen abgeschieden werden. Der Gehalt dieser Trägerkatalysatoren an den katalytisch aktiven Metallen ist prinzipiell für das Gelingen des erfindungsgemäßen Verfahrens nicht kritisch. Im Allgemeinen führen höhere Gehalte an katalytisch aktiven Metallen dieser Trägerkatalysatoren zu höheren Raum-Zeit-Umsätzen als niedrigere Gehalte. Im Allgemeinen werden Trägerkatalysatoren verwendet, deren Gehalt an katalytisch aktiven Metallen im Bereich von 0,1 bis 90 Gew. -%, vorzugsweise im Bereich von 0,5 bis 40 Gew. -% bezogen auf das Gesamtgewicht des Katalysators, liegt. Da sich diese Gehaltsangaben auf den gesamten Katalysator inklusive Trägermaterial beziehen, die unterschiedlichen Trägermaterialien jedoch sehr unterschiedliche spezifische Gewichte und spezifische Oberflächen haben, ist es auch denkbar, dass diese Angaben auch unter- oder überschritten werden können, ohne dass sich dies nachteilig auf das Ergebnis des erfindungsgemäßen Verfahrens auswirkt. Selbstverständlich können auch mehrere der katalytisch aktiven Metalle auf dem jeweiligen Trägermaterial aufgebracht sein. Weiterhin können die katalytisch aktiven Metalle beispielsweise nach dem Verfahren von DE-OS 25 19 817 oder EP 0 285 420 A1 auf den Träger aufgebracht werden.

In den Katalysatoren gemäß den vorgenannten Schriften liegen die katalytisch aktiven Metalle als Legierung vor, die durch thermische Behandlung und/oder Reduktion der z. B. durch Tränkung mit einem Salz oder Komplex der zuvor genannten Metalle, erzeugt werden.

Sowohl die Aktivierung der Fällungskatalysatoren als auch der Trägerkatalysatoren kann auch in situ zu Beginn der Reaktion durch den anwesenden Wasserstoff erfolgen. Bevorzugt werden diese Katalysatoren vor ihrer Verwendung separat aktiviert.

Als Trägermaterialien können im Allgemeinen die Oxide des Aluminiums und Titans, Zirkoniumdioxid, Siliciumdioxid, Tonerden wie beispielsweise Montmorillonite, Silikate wie beispielsweise Magnesium-oder Aluminiumsilikate, Zeolithe wie beispielsweise der Strukturtypen ZSM-5 oder ZSM-10, oder Aktivkohle verwendet werden. Bevorzugte Trägermaterialien sind Aluminiumoxide, Titandioxide, Siliciumdioxid, Zirkoniumdioxid und Aktivkohle. Selbstverständlich können auch Mischungen verschiedener Trägermaterialien als Träger für im erfindungsgemäßen Verfahren verwendbare Katalysatoren dienen.

Der mindestens eine heterogene Katalysator kann beispielsweise als Suspensionskatalysator und/oder als Festbettkatalysator eingesetzt werden.

Wird beispielsweise im Rahmen des erfindungsgemäßen Verfahrens die Hydrierung mit mindestens einem Suspensionskatalysator durchgeführt, so wird bevorzugt in mindestens einem Rührreaktor, oder in mindestens einer Blasensäule oder in mindestens einer gepackten Blasensäule oder in einer Kombination aus zwei oder mehr gleichen oder unterschiedlichen Reaktoren hydriert.

Der Begriff "unterschiedliche Reaktoren" bezeichnet vorliegend sowohl unterschiedliche Reaktortypen als auch Reaktoren der gleichen Art, die sich beispielsweise durch ihre Geometrie wie beispielsweise ihrem Volumen und/oder ihrem Querschnitt und/oder durch die Hydrierbedingungen in den Reaktoren unterscheiden.

Wird beispielsweise im Rahmen des erfindungsgemäßen Verfahrens die Hydrierung mit mindestens einem fest angeordneten Katalysator durchgeführt, so wird bevorzugt mindestens ein Rohrreaktor wie beispielsweise mindestens ein Schachtreaktor und/oder mindestens ein Rohrbündelreaktor verwendet, wobei ein einzelner Reaktor in Sumpf- oder Rieselfahrweise betrieben werden kann. Bei Verwendung von zwei oder mehr Reaktoren kann mindestens einer in Sumpffahrweise und mindestens einer in Rieselfahrweise betrieben werden.

Gemäß einer bevorzugten Ausführungsform des erfindungsgemäßen Verfahrens wird der für die Hydrierung eingesetzte mindestens eine Katalysator aus dem Produktgemisch der Hydrierung abgetrennt. Diese Abtrennung kann in Abhängigkeit vom eingesetzten Katalysator gemäß jeder geeigneten Verfahrensführung erfolgen.

Da die Anwesenheit homogener Hydrierkatalysatoren die Umsetzung des cyclischen Olefins der Formel I mit Distickstoffmonoxid nicht beeinträchtigen, kann die Abtrennung des Katalysators aus Rückführströmen auch unterbleiben.

Wird als Katalysator bei der Hydrierung beispielsweise ein heterogener Katalysator als Suspensionskatalysator eingesetzt, so wird dieser im Rahmen der vorliegenden Erfindung bevorzugt durch mindestens einen Filtrationsschritt abgetrennt. Der derart abgetrennte Katalysator kann in die Hydrierung rückgeführt werden oder mindestens einem beliebigen anderen Verfahren zugeführt werden. Ebenso ist es möglich, den Katalysator aufzuarbeiten, um beispielsweise das in dem Katalysator enthaltene Metall zurückzugewinnen.

Wird als Katalysator bei der Hydrierung beispielsweise ein homogener Katalysator eingesetzt, so wird dieser im Rahmen der vorliegenden Erfindung bevorzugt durch mindestens einen Destillationsschritt abgetrennt. Im Rahmen dieser Destillation können eine oder zwei oder mehr Destillationskolonnen verwendet werden. Der derart abgetrennte Katalysator kann in die Hydrierung rückgeführt werden oder einem beliebigen anderen Verfahren zugeführt werden. Ebenso ist es möglich, den Katalysator aufzuarbeiten, um beispielsweise das in dem Katalysator enthaltene Metall zurückzugewinnen.

Vor dem Einsatz in einem beliebigen Verfahren wie beispielsweise vor der Rückführung in das erfindungsgemäße Verfahren kann sowohl der mindestens eine homogene als auch der mindestens eine heterogene Katalysator, sollte dies erforderlich sein, durch mindestens ein geeignetes Verfahren regeneriert werden.

Die Wärmeabfuhr kann bei dem erfindungsgemäß verwendeten Reaktor intern beispielsweise über Kühlschlangen und/oder extern beispielsweise über mindestens einen Wärmetauscher abgeführt werden. Wird beispielsweise bevorzugt mindestens ein Rohrreaktor zur Hydrierung eingesetzt, so wird bevorzugt die Reaktion über äußeren Umlauf gefahren, in dem die Wärmeabführung integriert ist.

Wird gemäß einer bevorzugten Ausführungsform des erfindungsgemäßen Verfahrens die Hydrierung kontinuierlich durchgeführt, werden weiter bevorzugt mindestens zwei Reaktoren, weiter bevorzugt mindestens zwei Rohrreaktoren, weiter bevorzugt mindestens zwei seriell gekoppelte Rohrreaktoren und insbesondere bevorzugt zwei seriell gekoppelte Rohrreaktoren eingesetzt. Die Hydrierbedingungen in den eingesetzten Reaktoren können jeweils gleich oder unterschiedlich sein und liegen jeweils in den oben beschriebenen Bereichen.

Wird die Hydrierung an mindestens einem suspendierten Katalysator durchgeführt, liegt die Verweilzeit im Allgemeinen im Bereich von 0,5 bis 50 h, bevorzugt im Bereich von 1 bis 30 h und besonders bevorzugt im Bereich von 1,5 bis 25 h. Dabei ist es unerheblich, ob erfindungsgemäß ein Reaktor oder mindestens 2 in Serie geschaltete Reaktoren eingesetzt werden. Für sämtliche dieser Ausführungsformen liegt die Gesamtverweilzeit in den oben angegebenen Bereichen.

Wird im Rahmen des erfindungsgemäßen Verfahrens die Hydrierung in kontinuierlicher Fahrweise an mindestens einem fest angeordneten Katalysator durchgeführt, so liegt die Verweilzeit im Allgemeinen im Bereich von 0,1 bis 20 h, bevorzugt im Bereich von 0,2 bis 15 h und besonders bevorzugt im Bereich von 0,3 bis 10 h. Dabei ist es unerheblich, ob erfindungsgemäß ein Reaktor oder mindestens 2 in Serie geschaltete Reaktoren eingesetzt werden. Für sämtliche dieser Ausführungsformen liegt die Gesamtverweilzeit in den oben angegebenen Bereichen.

Der Wasserstoffdruck liegt bei der Hydrierung im Allgemeinen im Bereich von 1 bis 325 bar, bevorzugt im Bereich von 1,5 bis 200 bar, stärker bevorzugt im Bereich von 2 bis 100 bar und insbesondere bevorzugt im Bereich von 2,5 bis 50 bar.

Die Hydriertemperatur liegt im Allgemeinen im Bereich von 0 bis 250 °C, bevorzugt im Bereich von 20 bis 200 °C, beispielsweise im Bereich von 30 bis 180 °C, stärker bevorzugt im Bereich von 30 bis 150°C, besonders bevorzugt im Bereich von 40 bis 170 °C und insbesondere bevorzugt im Bereich von 40 bis 140 °C.

Aus dem bei der Hydrierung erhaltenen Produktgemisch isoliert man das gesättigte Keton der Formel III auf eine geeignete Weise, vorzugsweise durch Destillation. Dabei wird das gesättigte Keton der Formel III von Hochsiedem, die im Wesentlichen aus Diketon und Zersetzungsprodukten bestehen, und Leichtsiedem, die im Wesentlichen aus nicht umgesetztem cyclischen Olefin der Formel I bestehen, abgetrennt. Die Isolierung und Reinigung des gesättigten Ketons der Formel III lassen sich sowohl in einer, als auch in zwei oder drei Kolonnen durchführen. Es ist auch möglich, mehr Kolonnen einzusetzen, dies ist jedoch in der Regel nicht notwendig.

Vorzugsweise leitet man das Produktgemisch zuerst in den mittleren Bereich einer (ersten) Destillationskolonne mit Einbauten. Hierfür lässt sich jede beliebige Destillationskolonne verwenden. Unter dem "mittleren Bereich" einer Destillationskolonne versteht man den Bereich zwischen Kopf und Sumpf, also den Seitenzulauf, der Destillationskolonne.

Als Einbauten sind jegliche dem Fachmann bekannte Einbauten einsetzbar. Bevorzugte Einbauten sind ausgewählt aus der Gruppe Füllkörper wie Pall-Ringe und Raschig-Ringe, strukturierte Packungen aus Blech wie Mellapak 250 von Sulzer Ltd. (Winterthur/Schweiz), Montz (Hilden/Deutschland) und Koch-Glitsch (Wichita, KS/USA) und strukturierte Packungen aus Metallgewebe wie Sulzer BX (X3) von Sulzer Ltd. (Winterthur/Schweiz), Montz (Hilden/Deutschland) und Koch-Glitsch (Wichita, KS/USA).

Wird die fraktionierte Destillation nur in einer Kolonne durchgeführt, so wird vorzugsweise eine Trennwandkolonne eingesetzt. Dabei werden die leicht siedenden Verunreinigungen über Kopf der Kolonne und die schwer siedenden Verunreinigungen über Sumpf der Kolonne abgezogen. Der am Sumpf der Kolonne abgezogene Strom wird erst mit Hilfe eines Verdampfers verdampft. Die verdampfbaren Anteile werden dann in die Kolonne zurückgeführt, während die nicht verdampfbaren Anteile, bei denen es sich um die schwer siedenden Verunreinigungen handelt, ausgeschleust werden. Entsprechend wird mit dem am Kopf der Kolonne entnommenen Strom verfahren. Dieser wird zunächst in einem Kondensator kondensiert und teilweise in die Kolonne zur weiteren Auftrennung rückgeführt wird. Das gewünschte Wertprodukt wird auf der dem Zulauf gegenüber liegenden Seite der Trennwand im oberen Bereich der Kolonne, d. h. unterhalb des Kopfes der Kolonne, entnommen.

Werden zwei Kolonnen miteinander verschalten, werden die leicht siedenden Verunreinigungen über Kopf der ersten und/oder der zweiten Kolonne und die schwer siedenden Verunreinigungen über Sumpf der ersten und/oder der zweiten Kolonne abgetrennt. Es hat sich als vorteilhaft erwiesen, die leicht siedenden Verunreinigungen über Kopf der ersten Kolonne abzutrennen.

Der von leicht siedenden Verunreinigungen befreite Strom wird über Sumpf der ersten Kolonne ausgeschleust. Der über Sumpf der Kolonne ausgeschleuste Strom wird nun im mittleren Bereich in die zweite Kolonne eingeleitet. Die schwer siedenden Verunreinigungen werden über den Sumpf der Kolonne entnommen und ausgeschleust. Das gewünschte Wertprodukt, wird über Kopf der zweiten Kolonne entnommen.

Die Leichtsieder, die im Wesentlichen aus nicht umgesetztem cyclischen Olefin der Formel I bestehen, können mit Vorteil zumindest teilweise in den Schritt (a) des erfindungsgemäßen Verfahrens zurückgeführt werden.

Die Erfindung wird durch die folgenden Beispiele näher veranschaulicht.

### Beispiel 1: Synthese von 14-Methyl-bicyclo[10.3.0]pentadecen[1(12)]

### Schritt a): Alkylierung von Cyclododecanon mit Methallylchlorid

1458,4 g Cyclododecanon werden in 1440 g Toluol gelöst und mit 30,4 g Tetrabutylammoniumiodid versetzt. 1920 g Natronlauge (50%ig) werden zugegeben und die zweiphasige Mischung unter kräftigem Rühren auf 90 °C erhitzt. Über einen Zeitraum von 1 Stunde werden dann 1087,2 g Methallylchlorid zugetropft. Nach vollständiger Zugabe wird für 5 h bei 90 °C nachgerührt. Man lässt auf 60 °C abkühlen und gibt 1500 ml Wasser zu. Die Phasen werden getrennt und die organische Phase zunächst mit 2000 ml Wasser, dann mit 2000 ml 10 %iger H₂SO₄ und dann mit 2000 ml 10%iger Natronlauge gewaschen.

Alle Leichtsieder werden abdestilliert, anschließend wird das Produkt durch Rektifikation gereinigt. 2-(2-Methallyl)-cyclododecanon geht bei 10 mbar, 164-165 °C über. Es wird eine Ausbeute von 73% erhalten.

### Schritt b): Gasphasencyclisierung

70 ml (42,4g) Aluminiumoxid-Katalysator (Stränge 1,5 mm, D10-10 von BASF SE, Deutschland, calciniert bei 500 °C für 5 h unter Stickstoff) werden in eine Gasphasenapparatur mit einem Durchmesser von 1 cm und einer Länge von 30 cm, die durch eine elektrische Heizwendel beheizt ist, eingebaut. Bei einer Reaktionstemperatur von 280 °C bis 330 °C werden 10g/h 2-(2-Methallyl)-cyclododecanon (Verdampfungstemperatur 290°C) und 20 Nl/h Stickstoff als Gasstrom auf die Apparatur gefahren. Über einen Zeitraum von 190 h können bei einer zugefahrenen Menge von 1905 g Startmaterial 1755 g Cyclisierungsprodukt mit einem Gehalt von 72,3% des Hauptisomers (14-Methyl-bicyclo[10.3.0]pentadeca-1,(12),13-dien) und 13,8% an Nebenisomeren erhalten werden. Dieses Material wird ohne weitere Aufreinigung in die Folgestufe eingesetzt.

### Schritt c): Dien-Hydrierung:

150 g Rohaustrag aus der Gasphasenzyklisierung und 1,3 g Pd/C-Katalysator (∼5% Pd, ~50% wasserfeucht) werden in einem 300ml Autoklaven mit Schrägblattrührer vorgelegt. Der Autoklav wird mit H₂ gespült und anschließend das Reaktionsgemisch auf 100°C erhitzt. Bei Erreichen dieser Temperatur werden 5 bar H₂ aufgepresst und die Reaktion gestartet. Der H₂-Druck wird im Verlauf der Reaktion konstant bei 5 bar gehalten. Nach dem Ende der Reaktion wird der Autoklav abgekühlt, entspannt und der Katalysator abfiltriert. Der Katalysator kann ohne Aktivitätsverlust in eine erneute Hydrierung zurückgeführt werden. Nach 8h Reaktionszeit liegt der Dien-Umsatz bei 99,8% und die Selektivität bezogen auf 14-Methyl-bicyclo[10.3.0]pentadecen[1(12)] (la), das Hauptisomer des Produkts, bei 94,9%. Das so erhaltene Rohprodukt, mit einem Gehalt von 83,3 % Hauptisomer und 7,8 % Nebenkomponenten, wird ohne weitere Aufreinigung in der Folgestufe eingesetzt.

### Beispiel 2: Oxidation von (la) mit N₂O:

Die Reaktion wurde in einem Rohrreaktor mit einem Durchmesser von 1,7 mm und einem Gesamtvolumen von 210 ml durchgeführt. Das Rohr war zur Thermostatisierung mit einem Doppelmantel versehen über dem einen Wärmeträgeröl zirkuliert wurde dessen Temperatur mit einem externen Thermostat auf 280°C reguliert wurde. Der Reaktionsdruck wurde mit einem Druckhalteventil am Reaktorausgang auf 270 bar eingestellt. Die Edukte wurden mit zwei Dosierpumpen kontinuierlich zum Reaktor dosiert. Durch die erste Pumpe wurde flüssiges N₂O (Linde, 2.5, 25 g/h) und durch die zweite Pumpe das Olefin (Ia) (150 g/h). Der Versuch lief insgesamt 4 Stunden. Der Reaktoraustrag nach der Entspannung wurde abgekühlt, gesammelt und mit GC analysiert. Der Umsatz von Ia betrug 10,3%. Die Selektivität zu den gewünschten Produkten (3-Methylcyclopentadec-5-enon und 3-Methylcyclopentadec-4-enon jeweils als Gemisch der cis- und trans-Isomere, dieses Gemisch wird auch als Muscenon bezeichnet) betrug 50,5%.

### Beispiel 3: Hydrierung des Oxidationsaustrags:

1380 g Rohaustrag aus Beispiel 2 (enthaltend 4,7 % Muscenon, 74,2 % 14-Methyl-bicyclo[10.3.0]pentadecen[1 (12)], 4,3 % Isomere von 14-Methyl-bicyclo[10.3.0]pentadecen[1 (12)] und 1,6 % Diketone) und 11,0 g Pd/C-Katalysator (∼5% Pd, ~50% wasserfeucht) werden in einem 2,5L Autoklaven mit dreistufigem Schrägblattrührer vorgelegt. Der Autoklav wird mit H₂ gespült und anschließend das Reaktionsgemisch auf 100°C erhitzt. Bei Erreichen dieser Temperatur werden 10 bar H₂ aufgepresst und die Reaktion gestartet. Der H₂-Druck wird im Verlauf der Reaktion konstant bei 10 bar gehalten. Nach dem Ende der Reaktion wird der Autoklav abgekühlt, entspannt und der Katalysator abfiltriert. Nach 12h Reaktionszeit liegt der Muscenon-Umsatz bei 94,3% und die Selektivität zu Muscon bei 94,1%. Das so erhaltene Rohprodukt setzt sich aus 4,8 % Muscon, 73,9 % 14-Methyl-bicyclo[10.3.0]-pentadecen[1(12)], 4,5 % Isomere von 14-Methyl-bicyclo[10.3.0]pentadecen[1(12)] und 1,6 % Diketone zusammen und kann entweder destillativ getrennt oder ohne weitere Aufarbeitung in die Oxidation zurückgeführt werden.

## Patentansprüche

1. Verfahren zur Herstellung von Ketonen der Formel II, 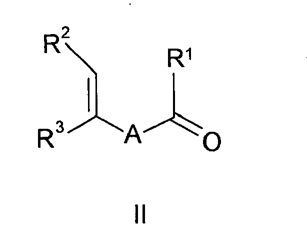 worin
A für C₂-C₁₂-Alkandiyl steht, das gegebenenfalls durch 1 bis 5 C₁-C₆-Alkylgruppen substituiert ist,
R¹ und R² unabhängig voneinander für C₁-C₆-Alkyl stehen, oder R¹ und R² gemeinsam für C₃-C₁₀-Alkandiyl steht, das gegebenenfalls durch 1 bis 5 C₁-C₆-Alkylgruppen substituiert ist, und
R³ für Wasserstoff oder C₁-C₆-Alkyl steht;
wobei man
(a) ein cyclisches Olefin der Formel I 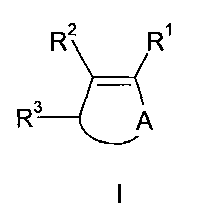 mit Distickstoffmonoxid zum Keton der Formel II umsetzt.

2. Verfahren nach Anspruch 1, wobei man außerdem
(b) das Keton der Formel II zum gesättigten Keton der Formel III hydriert

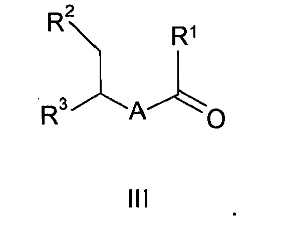

3. Verfahren nach Anspruch 2, wobei man
(i) das cyclische Olefin der Formel I unter Teilumsatz mit Distickstoffmonoxid zu einem ersten Gemisch umsetzt, das nicht umgesetztes cyclisches Olefin der Formel I und Keton der Formel II umfasst, und
(ii) das erste Gemisch unter Bedingungen hydriert, unter denen das Keton der Formel II zum gesättigten Keton der Formel III hydriert wird und das cyclische Olefin der Formel I im Wesentlichen nicht hydriert wird, wobei man ein zweites Gemisch erhält, das nicht umgesetztes cyclisches Olefin der Formel I und gesättigtes Keton der Formel III umfasst.

4. Verfahren nach Anspruch 3, wobei man zumindest einen Teil des zweiten Gemisches zum Schritt (i) zurückführt.

5. Verfahren nach Anspruch 4, wobei man die Schritte (i) und (ii) wiederholt, bis ein gewünschter Gesamtumsatz des cyclischen Olefins der Formel I erreicht ist.

6. Verfahren nach Anspruch 4, wobei man einen Teil des zweiten Gemisches mit frischem cyclischen Olefin der Formel I versetzt und zum Schritt (i) zurückführt und einen Teil des zweiten Gemisches abzieht und gesättigtes Keton der Formel III daraus isoliert.

7. Verfahren nach einem der Ansprüche 3 bis 6, wobei man aus dem zweiten Gemisch das gesättigte Keton der Formel III durch Destillation isoliert.

8. Verfahren nach Anspruch 2, wobei man
(i) das cyclische Olefin der Formel I unter Teilumsatz mit Distickstoffmonoxid zu einem ersten Gemisch umsetzt, das nicht umgesetztes cyclisches Olefin der Formel I und Keton der Formel II umfasst, und
(ii) aus dem ersten Gemisch nicht umgesetztes cyclisches Olefin der Formel I abtrennt, wobei man einen Rückstand erhält, der ungesättigtes Keton der Formel II umfasst, und den Rückstand hydriert, wobei man einen hydrierten Rückstand erhält, der gesättigtes Keton der Formel III umfasst.

9. Verfahren nach Anspruch 8, wobei man das abgetrennte nicht umgesetzte cyclische Olefin der Formel I zumindest teilweise in den Schritt (i) zurückführt.

10. Verfahren nach einem der Ansprüche 3 bis 9, wobei der Teilumsatz 20 Mol-% oder weniger, bezogen auf das cyclische Olefin der Formel I, beträgt.

11. Verfahren nach einem der vorhergehenden Ansprüche, wobei man die Umsetzung mit Distickstoffmonoxid in Abwesenheit eines externen Lösungsmittels durchführt.

12. Verfahren nach einem der Ansprüche 2 bis 11, wobei man die Hydrierung in Abwesenheit eines externen Lösungsmittels durchführt.

13. Verfahren nach einem der vorhergehenden Ansprüche, wobei man die Umsetzung mit Distickstoffmonoxid unter einem Druck durchführt, unter dem Distickstoffmonoxid in kondensierter Phase vorliegt.

14. Verfahren nach einem der vorhergehenden Ansprüche, wobei man die Hydrierung in Gegenwart eines heterogenen Hydrierkatalysators durchführt.

15. Verfahren nach einem der Ansprüche 2 bis 14 zur Herstellung von (±)-3-Methylcyclopentadecanon der Formel IIIa, 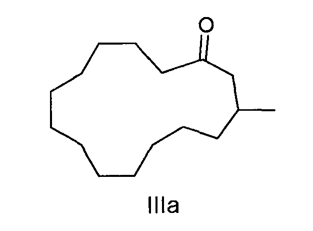 wobei man 14-Methyl-bicyclo[10.3.0]pentadecen[1(12)] der Formel la als cyclisches Olefin der Formel I einsetzt

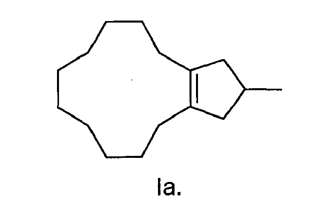

## Claims

1. A process for preparing ketones of the formula II, 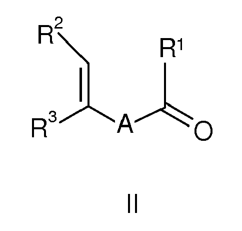 where
A is C₂-C₁₂-alkanediyl which is optionally substituted by from 1 to 5 C₁-C₆-alkyl groups,
R¹ and R² are each, independently of one another, C₁-C₆-alkyl or R¹ and R² together form C₃-C₁₀-alkanediyl which is optionally substituted by from 1 to 5 C₁-C₆-alkyl groups and
R³ is hydrogen or C₁-C₆-alkyl;
wherein
(a) a cyclic olefin of the formula I 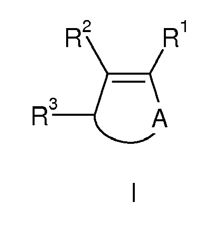 is reacted with dinitrogen monoxide to give the ketone of the formula II.

2. The process according to claim 1, wherein, in addition,
(b) the ketone of the formula II is hydrogenated to the saturated ketone of the formula III

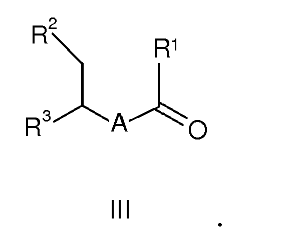

3. The process according to claim 2, wherein
(i)the cyclic olefin of the formula I is reacted to a partial conversion with dinitrogen monoxide to give a first mixture which comprises unreacted cyclic olefin of the formula I and ketone of the formula II and
(ii) the first mixture is hydrogenated under conditions under which the ketone of the formula II is hydrogenated to the saturated ketone of the formula III and the cyclic olefin of the formula I is essentially not hydrogenated, giving a second mixture which comprises unreacted cyclic olefin of the formula I and saturated ketone of the formula III.

4. The process according to claim 3, wherein at least part of the second mixture is recirculated to step (i).

5. The process according to claim 4, wherein steps (i) and (ii) are repeated until a desired total conversion of the cyclic olefin of the formula I has been achieved.

6. The process according to claim 4, wherein part of the second mixture is admixed with fresh cyclic olefin of the formula I and recirculated to step (i) and part of the second mixture is taken off and saturated ketone of the formula III is isolated therefrom.

7. The process according to any of claims 3 to 6, wherein the saturated ketone of the formula III is isolated from the second mixture by distillation.

8. The process according to claim 2, wherein
(i) the cyclic olefin of the formula I is reacted to a partial conversion with dinitrogen monoxide to give a first mixture which comprises unreacted cyclic olefin of the formula I and ketone of the formula II and
(ii) unreacted cyclic olefin of the formula I is separated off from the first mixture to give a residue which comprises unsaturated ketone of the formula II and the residue is hydrogenated to give a hydrogenated residue which comprises saturated ketone of the formula III.

9. The process according to claim 8, wherein the unreacted cyclic olefin of the formula I which has been separated off is at least partly recirculated to step (i).

10. The process according to any of claims 3 to 9, wherein the partial conversion is 20 mol% or less, based on the cyclic olefin of the formula I.

11. The process according to any of the preceding claims, wherein the reaction with dinitrogen monoxide is carried out in the absence of an external solvent.

12. The process according to any of claims 2 to 11, wherein the hydrogenation is carried out in the absence of an external solvent.

13. The process according to any of the preceding claims, wherein the reaction with dinitrogen monoxide is carried out under a pressure under which dinitrogen monoxide is present in a condensed phase.

14. The process according to any of the preceding claims, wherein the hydrogenation is carried out in the presence of a heterogeneous hydrogenation catalyst.

15. The process according to any of claims 2 to 14 for preparing (±)-3-methylcyclopentadecanone of the formula IIIa, 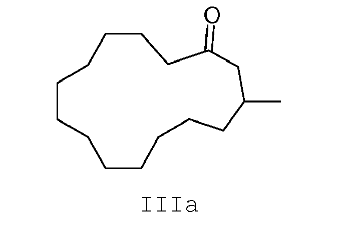 wherein 14-methylbicyclo[10.3.0]pentadecene[1(12)] of the formula Ia is used as cyclic olefin of the formula I

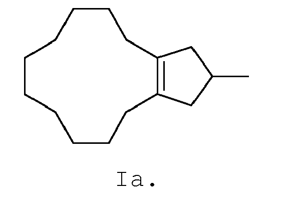

## Revendications

1. Procédé pour la préparation de cétones de formule II, 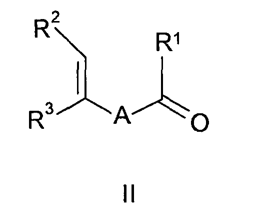 où
A représente C₂-C₁₂-alcanediyle, qui est le cas échéant substitué par 1 à 5 groupes C₁-C₆-alkyle,
R¹ et R² représentent, indépendamment l'un de l'autre, C₁-C₆-alkyle, ou R¹ et R² représentent ensemble C₃-C₁₀-alcanediyle, qui est le cas échéant substitué par 1 à 5 groupes C₁-C₆-alkyle, et
R³ représente hydrogène ou C₁-C₆-alkyle,
dans lequel on transforme
(a) une oléfine cyclique de formule I 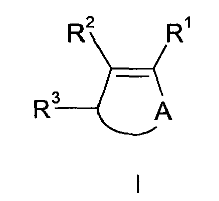 avec du protoxyde d'azote en cétone de formule II.

2. Procédé selon la revendication 1, dans lequel, en outre
(b) on hydrogène la cétone de formule II en cétone saturée de formule III

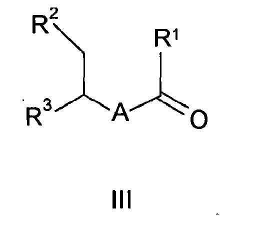

3. Procédé selon la revendication 2, dans lequel
(i) on transforme l'oléfine cyclique de formule I, sous une conversion partielle, avec du protoxyde d'azote en un premier mélange, qui contient l'oléfine cyclique de formule I non transformée et la cétone de formule II, et
(ii) on hydrogène le premier mélange dans des conditions dans lesquelles la cétone de formule II est hydrogénée en cétone saturée de formule III et l'oléfine cyclique de formule I n'est pratiquement pas hydrogénée, avec obtention d'un deuxième mélange qui comprend l'oléfine cyclique de formule I non transformée et la cétone saturée de formule III.

4. Procédé selon la revendication 3, dans lequel on recycle au moins une partie du deuxième mélange dans l'étape (i).

5. Procédé selon la revendication 4, dans lequel les étapes (i) et (ii) sont répétées jusqu'à ce qu'on atteigne une conversion totale souhaitée de l'oléfine cyclique de formule I.

6. Procédé selon la revendication 4, dans lequel on additionne une partie du deuxième mélange d'oléfine cyclique de formule I fraîche et on la recycle dans l'étape (i) et on soutire une partie du deuxième mélange et on isole la cétone saturée de formule III de celle-ci.

7. Procédé selon l'une quelconque des revendications 3 ou 6, dans lequel on isole, à partir du deuxième mélange, la cétone saturée de formule III par distillation.

8. Procédé selon la revendication 2, dans lequel
(i) on transforme l'oléfine cyclique de formule I, sous une conversion partielle, avec du protoxyde d'azote en un premier mélange, qui contient l'oléfine cyclique de formule I non transformée et la cétone de formule II, et
(ii) on sépare du premier mélange l'oléfine cyclique de formule I non transformée, avec obtention d'un résidu qui comprend la cétone insaturée de formule II et on hydrogène le résidu, avec obtention d'un résidu hydrogéné qui comprend la cétone saturée de formule III.

9. Procédé selon la revendication 8, dans lequel l'oléfine cyclique de formule I non transformée séparée est recyclée au moins partiellement dans l'étape (i).

10. Procédé selon l'une quelconque des revendications 3 à 9, dans lequel la conversion partielle est de 20% en mole ou moins, par rapport à l'oléfine cyclique de formule I.

11. Procédé selon l'une quelconque des revendications précédentes, dans lequel la transformation avec du protoxyde d'azote est réalisée en l'absence d'un solvant externe.

12. Procédé selon l'une quelconque des revendications 2 à 11, dans lequel l'hydrogénation est réalisée en l'absence d'un solvant externe.

13. Procédé selon l'une quelconque des revendications précédentes, dans lequel la transformation avec du protoxyde d'azote est réalisée sous une pression à laquelle le protoxyde d'azote se trouve en phase condensée.

14. Procédé selon l'une quelconque des revendications précédentes, dans lequel on réalise l'hydrogénation en présence d'un catalyseur d'hydrogénation hétérogène.

15. Procédé selon l'une quelconque des revendications 2 à 14 pour la préparation de (±)-3-méthylcyclopentadécanone de formule IIIa, 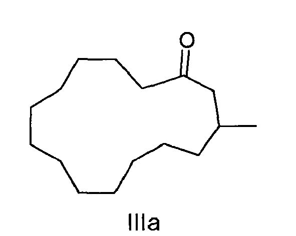 dans lequel on utilise du 14-méthylbicyclo[10,3,0]pentadécène[1(12)] de formule Ia comme oléfine cyclique de formule I
